# EUROPEAN PATENT SPECIFICATION

(11) **EP 2 241 289 B1**
(45) Date of publication and mention of the grant of the patent: **08.10.2014**
(21) Application number: 09700336.2
(22) Date of filing: 08.01.2009
(51) Int. Cl.: A61F 2/38

(54) **BEARING MEMBER INCLUDING A FLEXION, FOR AN ARTIFICIAL KNEE JOINT**
LAGERELEMENT MIT BIEGUNG, FÜR KÜNSTLICHES KNIEGELENK
ÉLÉMENT SUPPORT COMPRENANT UNE FLEXION, POUR ARTICULATION ARTIFICIELLE DU GENOU

(30) Priority: 08.01.2008 KR 20080002240
(43) Date of publication of application: 20.10.2010
(73) Proprietor: Corentec Inc., Chungcheongnam-do 330-822 (KR)
(72) Inventor: SUN, Doo-Hoon, Seoul 140-210 (KR); KIM, Yong-Sik, Seoul 137-040 (KR); KIM, Jung-Sung, Cheonan-si Chungcheongnam-do 331-230 (KR); KIM, Byung-Soo, Seoul 143-224 (KR); SEO, Jai-Gon, Seoul 135-230 (KR); CHO, Woo-Shin, Seoul 138-040 (KR); CHUNG, Hyun-Kee, Seoul 135-080 (KR); LEE, Myung-Chul, Seoul 110-460 (KR)
(74) Representative: Witte, Weller & Partner Patentanwälte mbB
(86) International application number: PCT/KR2009/000101
(87) International publication number: WO 2009/088235

(56) References cited:
- EP-A1- 1 159 938
- EP-A1- 1 440 675
- EP-A2- 1 336 395
- KR-A- 19980 701 639
- KR-A- 20000 028 553
- KR-B1- 100 314 509
- KR-B1- 100 776 079
- US-A1- 2005 096 747
- US-B1- 7 413 577

## Description

### [Technical Field]

The present invention relates generally to an artificial knee joint which can replace a natural knee joint. More specifically, this invention is directed to an artificial knee joint having a femur joint member which is attached to an end portion of the femur near the tibia, and a tibia joint member which is attached to an end portion of the tibia near the femur, wherein the artificial knee joint is designed to copy an actual knee movement by having the curvature of the front portion of a groove that is different from that of the rear portion of the groove, when viewing a bearing member between the femur and tibia joint members from the plane. In addition, seen in the lateral view, the bearing member is designed to have the curvatures of the front and rear portions different from each other. Therefore, the front portion is higher than and has a shorter curvature than the rear portion, thus preventing anterior dislocation, and the rear portion is lower than and has a longer curvature than the front portion so that more natural flection may occur in the rear portion.

### [Background Art]

Among the many joints in the body, the knee joint is the junction between the tibia and the femur. Because of the wearing of the knee joint, the aging of the bone tissue and accidents, the number of patients for whom recovery is impossible is gradually increasing. The knee joint is the joint which is located between the lower end of the femur, the upper end of the tibia, and the back of the patella, and functions to bend the leg backwards at the knee.

The back of the patella is covered by cartilage 4mm to 6mm thick. The patella moves up and down along the articular surface which is in front of the end portion of a thighbone (the femur) while the knee is bent or stretched (at the patellofemoral joint), thus improving the knee stretching force of the musculus quadriceps femoris. Pressure acting on the patellofemoral joint when a person walks on a flat ground is equal to half of his or her weight. When a person goes up the stairs, a force which is 3 times as great as his or her weight acts on the patellofemoral joint. Further, when a person sitting in a squatting position stands up, a force which is 8 times as great as his or her weight acts on the patellofemoral joint. An articular capsule extends from the edge of the lower end of the femur to the edge of the upper end of the tibia. In addition to the above components, the inner and outer collateral ligaments, the cruciate ligaments of knee in the articular capsule, and other strong ligaments strengthen the connection of the bones and limit the moving direction and range of the bones.

The symptoms that appear when the meniscus of the knee joint is damaged will now be described. Here, the meniscus of the knee joint is the cartilaginous tissue which is located between the femur and the tibia forming the knee joint. The meniscus is positioned between the articular cartilage to absorb shocks acting on the knee joint, supply nutrients to the articular cartilage, provide the joint with stability, allow the knee joint to move smoothly, and transmit the load imposed by the weight.

Generally, the meniscus includes a medial meniscus and a lateral meniscus. As for Europeans and Americans, the medial meniscus is larger in size and smaller in mobility than the lateral meniscus, so that the medial meniscus is easily damaged. However, it is known the lateral meniscus of Koreans is more heavily damaged than is the medial meniscus.

The damage to the meniscus is one type of damage frequently occurring in knee joints. The meniscus is frequently damaged during athletic sports, mountain climbing or daily life. This easily occurs when the bent knee is rotated, that is, when torsional force is applied to the knee joint. When excessive external force acts on the knee joint, a cruciate ligament or collateral ligament may be damaged and the tibia may be fractured.

Most symptoms of disease taking place in the patella do not reveal any noteworthy external injury, and are caused by structural and functional disorders of the patellofemoral joint. When the leg is bent abnormally outward or the foot is bent excessively outward, excessive force is repeatedly exerted on the patellofemoral joint, thus leading to the malacia of the articular cartilage. Even when the knee joint is not used for a lengthy period of time, the musculus quadriceps femoris may become weak, thus causing damage to the patella. When there are structural disorders of the patellofemoral joint, it is possible to wear an orthosis to stabilize the patella. Meanwhile, when the damage to the patellofemoral joint is severe, surgical procedures may be performed to replace the damaged joint with an artificial knee joint.

Recently, a surgical procedure for replacing a patient's joint which has been so severely damaged that it is impossible to recover with an artificial joint has been widely performed. Metal, ceramic or polyethylene is used for the motion part of the artificial joint, thus providing superior mechanical characteristics, reducing the coefficient of friction, and enhancing biocompatibility. Generally, the artificial knee joint includes a femur part, a tibia part, and a bearing part which is provided between the femur part and the tibia part and corresponds to cartilage. Here, the femur part and the tibia part are most commonly made of a metal alloy, while the cartilage part is made of polyethylene or the like. The tibia part is secured by an insertion part which is inserted into an end of the tibia near the knee joint. The insertion part is attached to the marrow of the tibia. However, if a load is repetitively applied to the knee joint, it is difficult for the artificial knee joint to achieve a sufficient effect, because of its structural defects. The femur part and the tibia part may be damaged by the continuous imposition of a load. Especially should the bearing part be broken, big problems occur.

A groove is formed on the upper surface of the bearing member, is in friction contact with the femur joint member, and enables the flection of the knee when the femur joint member is in contact with the groove and rolls. However, when the knee bends backwards as part of the flection of the knee, the tibia region tends to be adducted inward relative to the body. Further, when the knee is stretched out straight, the knee tends to bend slightly forward, and when it does so, the tibia region tends to be abducted slightly outward. As such, when the natural knee joint of the body is bent, adduction and abduction occur. However, conventional artificial knee joints cannot realize such a movement of the knee.

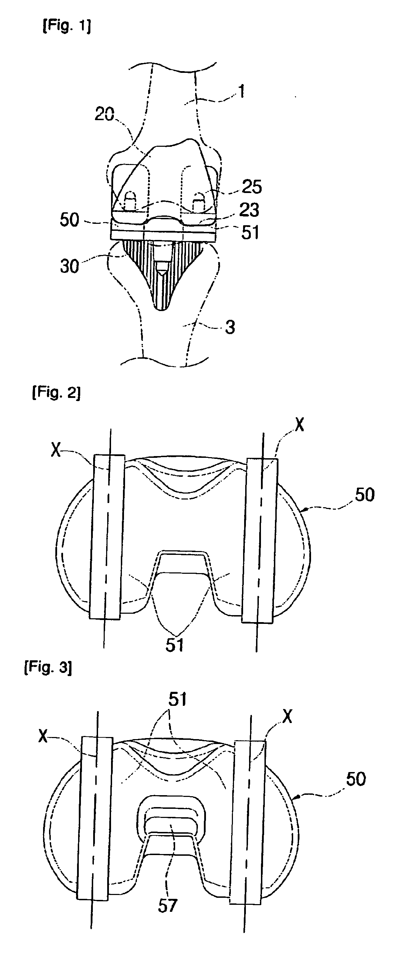

A conventional artificial knee joint is shown in FIGS. 1 and 2 and 3. FIG. 1 is an elevation illustrating the artificial knee joint which has been surgically implanted into the knee, FIG. 2 is a plan view illustrating a bearing member of a conventional CR type artificial knee joint, and FIG. 3 is a plan view illustrating a bearing member of a conventional PS type artificial knee joint. Here, the CR type joint is an artificial knee joint which is implanted in the state in which a posterior cruciate ligament is not removed. Meanwhile, the PS type joint is an artificial knee joint, in which a posterior cruciate ligament is removed and then is replaced with a post of the bearing member. When viewed from the plane, the groove on the upper surface of the bearing member of the conventional artificial knee joint is a straight groove in a longitudinal direction. Thus, when the femur joint member located at an upper position is in contact with the groove and rolls, a flection is performed linearly forwards and backwards. Therefore, an artificial knee joint having a bearing member which can perform the adduction and abduction of the tibia so as to achieve the more natural flection of the knee is required.

Further, in the case where the curvature of a front portion is the same as that of a rear portion when the bearing member is viewed from the side, if the femur joint member which is in contact with the upper portion of the bearing member and rolls is bent forward, anterior dislocation may occur. In contrast, when the femur joint member is bent backward, it is difficult to achieve a natural flection. Therefore, the groove is formed such that the curvature of the front portion is different from that of the rear portion when the bearing member is viewed from the side, thus preventing anterior dislocation and realizing a natural bending action.

US 2005/096747 A1, US 7 413 577 B1 which was published after the priority date of the present application, EP 1 440 675 A1, EP 1 159 938 A1 and EP 1 336 395 A2 disclose an artificial knee joint comprising a femoral component attached to a femur and a tibial component attached to a tibia and having two concavities on an upper surface thereof.

In US 2005/096747 A1, this document forming the basis for the preamble of claim 1, the femoral component has a medial condyle and a lateral condyle, and the tibial component defines a lateral concavity and a medial concavity. The lateral concavity has a different shape than the medial concavity, the medial and lateral concavities have first portions that are in articulating contact with the medial and lateral condyles of the femoral component during normal knee flexion, and the medial and lateral concavities have second portions that are at least partially separate from the first portions and are in articulating contact with the medial and lateral condyles of the femoral component during high knee flexion.

### [Disclosure]

### [Technical Problem]

Accordingly, the present invention has been made keeping in mind the above problems occurring in the prior art, and an object of the present invention is to provide an artificial knee joint including a flexion in a bearing member, having a femur joint member that is attached to an end portion of a femur near the tibia, a tibia joint member that is attached to an end portion of a tibia near the femur, and a bearing member that is positioned between the femur joint member and the tibia joint member. In the plan view of the bearing member between the femur and tibia joint members, the artificial knee joint member is designed to copy actual knee movement by having the curvature of a front portion of a groove that is different from the curvature of a rear portion of the groove.

Another object of the present invention is to provide an artificial knee joint including a flexion in a bearing member, in which, , when viewing the bearing member from the plane, a front portion of a groove has a curvature which allows the tibia region to be twisted at an angle when the knee is bent slightly forward, and a rear portion of the groove has a curvature which allows the tibia region to be twisted at an angle when the knee is bent backward, so as to perform a movement similar to the flection of the natural knee.

A further object of the present invention is to provide an artificial knee joint including a flexion in a bearing member, in which the curvature radius of a front portion of a groove of the bearing member is smaller than that of a rear portion so that the front portion twists at a larger angle, and the rear portion of the groove has a smaller curvature radius so that the rear portion twists at a smaller angle.

Yet another object of the present invention is to provide an artificial knee joint including a flexion in a bearing member, in which the curvature of a front portion is different from that of a rear portion when viewing the bearing member from the side, thus preventing anterior dislocation and enabling a natural flection.

Still another object of the present invention is to provide an artificial knee joint including a flexion in a bearing member, in which, in the side and sectional views of the bearing member, a front projecting part is formed to be high and have small curvature, thus preventing a femur joint member from being displaced forward in the artificial knee joint when the tibia region is slightly bent forward during the rotating movement of the knee, and a rear projecting part is formed to be low and have large curvature, thus improving a flection so as to permit the natural rotation of the knee when the tibia region is bent backward.

Another object of the present invention is to provide an artificial knee joint including a flexion in a bearing member, in which a rear projecting part of the bearing member may be formed to be low, thus preventing the femur region to which there is no femur joint member joined, from coming into contact with and damaging the rear projecting part, at the time of the roll back wherein the knee moves inwards at a deep angle.

### [Technical Solution]

In order to accomplish the above objects, the present invention provides a bearing member, comprising: a groove provided on an upper surface thereof and having a planar flexion to move a knee joint similarly to a real knee movement; and a side flexion including a side front flexion and a side rear flexion which have different curvature radii so as to more naturally move the knee joint, wherein the planar flexion comprises a planar rear flexion which is a contact portion when a knee is bent backward, and a planar front flexion which is a contact portion when the knee is stretched forward, characterized in that a curvature radius of the planar front flexion is smaller than a curvature radius of the planar rear flexion.

In an aspect of the invention, the bearing member may include a front projecting part and a rear projecting part being provided on the front and rear ends of the bearing member when viewed from the side.

In another aspect of the invention, a height of the front projecting part may be higher than a height of the rear projecting part, thus allowing a flection of the knee to be more naturally performed when the knee is bent, and preventing anterior dislocation of the femur joint member.

In still another aspect of the invention, the curvature radius of the side front flexion may be smaller than the curvature radius of the side rear flexion.

In order to accomplish the above objects, the present invention provides an artificial knee joint including a flexion in the bearing member mentioned above, having a femur joint member attached to an end portion of a femur, a tibia joint member attached to an end portion of a tibia, and the bearing member positioned between the femur joint member and the tibia joint member.

### [Advantageous Effects]

As described above, the present invention can accomplish the following effects by the above-mentioned technical solutions and the construction and operation to be mentioned later.

The present invention is advantageous in that the artificial knee joint includes a femur joint member that is attached to an end portion of a femur near the tibia, a tibia joint member that is attached to an end portion of a tibia near the femur, and a bearing member that is positioned between the femur joint member and the tibia joint member, and the artificial knee joint is designed to copy actual knee movement by having the curvature of the front portion of a groove that is different from the curvature of the rear portion of the groove, in the plan view of the bearing member between the femur and tibia joint members.

The present invention is advantageous in that, when viewing the bearing member from the plane, a front portion of a groove has a curvature which allows the tibia region to be twisted at an angle when the knee is bent slightly forward, and a rear portion of the groove has a curvature which allows the tibia region to be twisted at an angle when the knee is bent backward, so as to perform a movement similar to the flection of the natural knee.

According to the present invention, as the natural knee moves, the tibia region tends to be adducted inwards relative to the body when the knee is bent, and the tibia region tends to be abducted outwards relative to the body when the knee is stretched out straight. Thus, in order to provide a joint movement similar to a real knee movement, the curvature radius of a front portion of a groove of the bearing member is smaller than that of a rear portion so that the front portion twists at a larger angle, and the rear portion of the groove has a smaller curvature radius so that the rear portion twists at a smaller angle.

The present invention is advantageous in that the curvature of a front portion is different from that of a rear portion when viewing the bearing member from the side, thus preventing anterior dislocation and enabling a natural flection.

The present invention is advantageous in that, in the side and sectional views of the bearing member, a front projecting part is formed to be high and have small curvature, thus preventing a femur joint member from being displaced forward in the artificial knee joint when the tibia region is slightly bent forward during the rotating movement of the knee, and a rear projecting part is formed to be low and have large curvature, thus improving a flection so as to permit the natural rotation of the knee when the tibia region is bent backward.

The present invention is advantageous in that a rear projecting part of the bearing member may be formed to be low, thus preventing the femur region to which there is no femur joint member joined, from coming into contact with and damaging the rear projecting part, at the time of the roll back wherein the knee moves inwards at a deep angle.

### [Description of Drawings]

FIG. 1 is a view illustrating a conventional artificial knee joint;
FIG. 2 is a view illustrating grooves of a bearing member of a conventional CR type artificial knee joint;
FIG. 3 is a view illustrating grooves of a bearing member of a conventional PS type artificial knee joint;
FIG. 4 is a view illustrating grooves having curvature in a CR type bearing member according to the present invention;
FIG. 5 is a view illustrating grooves having curvature in a PS type bearing member according to the present invention;
FIG. 6 is a view illustrating the state in which the knee is bent in an artificial knee joint of the present invention;
FIG. 7 is a view illustrating a planar rear flexion in a bearing member of the present invention;
FIG. 8 is a view illustrating the state of the knee being stretched in the artificial knee joint of the present invention;
FIG. 9 is a view illustrating a planar front flexion in the bearing member of the present invention;
FIG. 10 is a view illustrating a side flexion in the bearing member of the present invention;
FIG. 11 is a view illustrating a side front projecting part in the bearing member of the present invention; and
FIG. 12 is a view illustrating a side rear projecting part in the bearing member of the present invention.

### [Best Mode]

Hereinafter, an artificial knee joint including a flexion in a bearing member according to the preferred embodiment of the present invention will be described in detail with reference to the accompanying drawings.

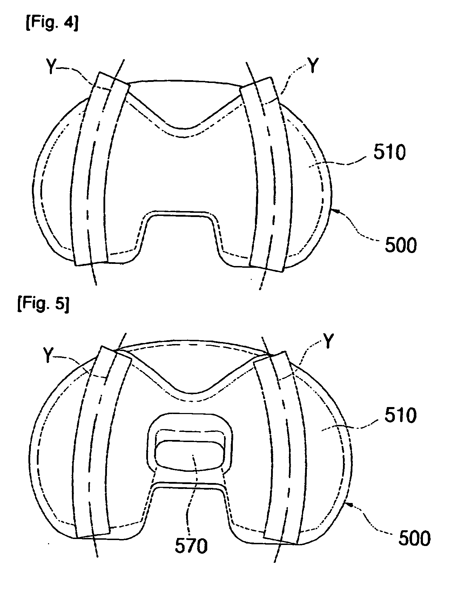

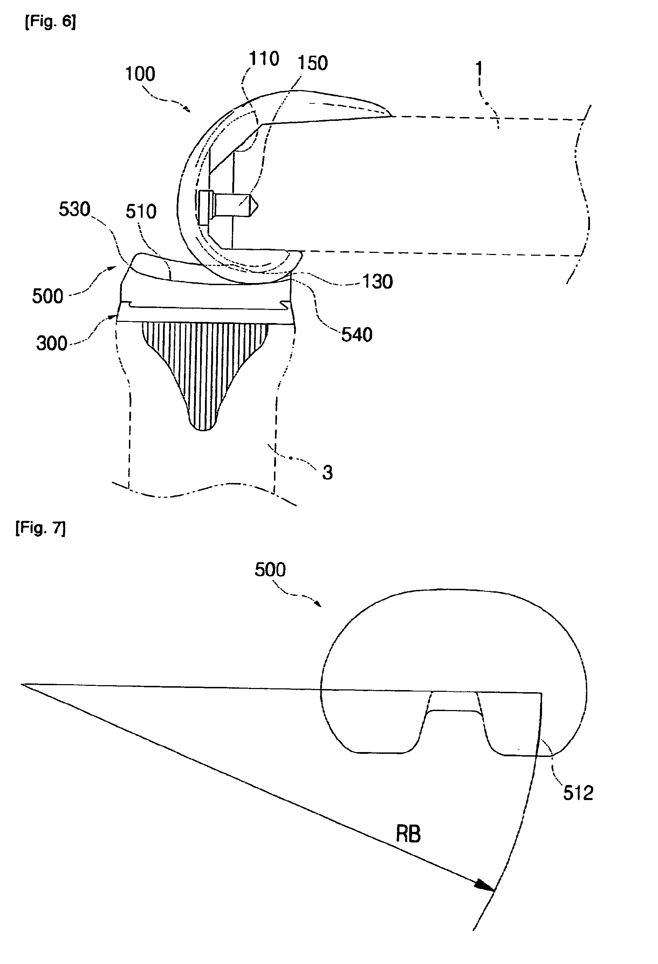

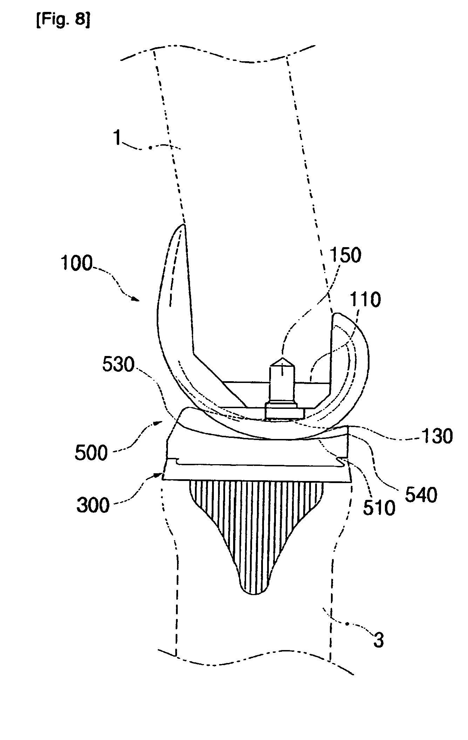

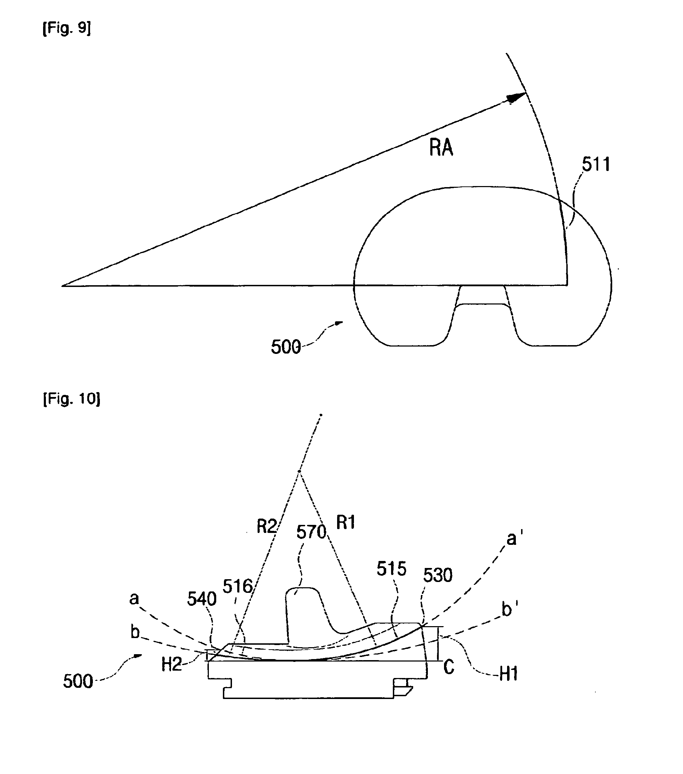

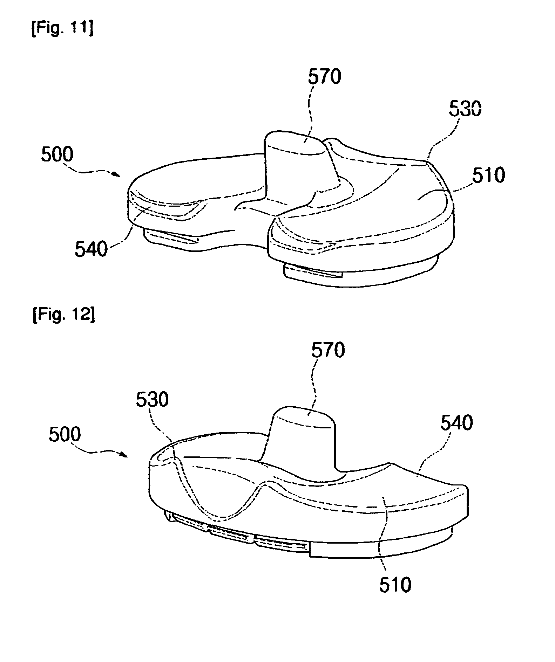

FIG. 1 is a view illustrating a conventional artificial knee joint, FIG. 2 is a view illustrating grooves of a bearing member of a conventional CR type artificial knee joint, FIG. 3 is a view illustrating grooves of a bearing member of a conventional PS type artificial knee joint, FIG. 4 is a view illustrating grooves having curvature in a CR type bearing member according to the present invention, FIG. 5 is a view illustrating grooves having curvature in a PS type bearing member according to the present invention, FIG. 6 is a view illustrating the state in which the knee is bent in an artificial knee joint of the present invention, FIG. 7 is a view illustrating a planar rear flexion in a bearing member of the present invention, FIG. 8 is a view illustrating the state of the knee being stretched in the artificial knee joint of the present invention, FIG. 9 is a view illustrating a planar front flexion in the bearing member of the present invention, FIG. 10 is a view illustrating a side flexion in the bearing member of the present invention, FIG. 11 is a view illustrating a side front projecting part in the bearing member of the present invention, and FIG. 12 is a view illustrating a side rear projecting part in the bearing member of the present invention.

First, the conventional artificial knee joint and the grooves 51 of the bearing member 50 will be described with reference to FIGS. 1 to 3. As shown in FIG. 1, the conventional artificial knee joint is implanted as follows. First, part of a contact portion of the femur 1 and the tibia 3 is cut, the femur joint member 20 and the tibia joint member 30 are joined to the contact portion and thereafter the bearing member 50 is placed between the femur joint member 20 and the tibia joint member 30 to facilitate rotation and forward and backward movement. The bearing member 50 has on its upper surface the grooves 51, so that the femur joint member 20 is in contact with and rotatably rolls along the grooves. In the plan view of the grooves 51 of the bearing member 50, the grooves 51 are formed in the direction of straight lines X in such a way as to be parallel to each other, as shown in FIGS. 2 and 3. As such, since the grooves 51 have the shape of the straight lines X, the knee moves merely in a linear direction when the femur joint member 20 located at an upper position rotatably rolls. FIG. 2 illustrates the artificial knee joint which is implanted with the posterior cruciate ligament being maintained, and illustrates a CR Type bearing member, and FIG. 3 illustrates a PS Type bearing member which is implanted in the state in which the posterior cruciate ligament is removed. The PS type bearing member 50 of FIG. 3 includes the post 57. Such a post 57 serves as the removed posterior cruciate ligament. However, when the knee actually bends or is stretched out, the knee may be adducted or abducted leftward or rightward. It is impossible for the conventional bearing members to simulate the movement of the natural knee because of the shape of the groove 51 of the bearing member 50, regardless of the CR type or PS type. Thus, the present invention provides an improved bearing member 500 so as to simulate the movement of the natural knee.

Before the bearing member 500 of the artificial knee joint according to the present invention is described with reference to FIGS. 4 and 5, respective components of the artificial knee joint according to the present invention will be described. Referring to FIGS. 6 to 8, the artificial knee joint of the present invention includes a femur joint member 100 which is attached to the lower portion of the femur 1, a tibia joint member 300 which is attached to the upper portion of the tibia 3, and a bearing member 500 which is provided between the femur joint member and the tibia joint member and serves as cartilage. The femur joint member 100 is in friction contact with the bearing member 500, and the bearing member is subjected to stress generated by load which is transmitted from the upper portion of the femur joint member. Further, the femur joint member is in contact with the bearing member, so that the tibia may move forward, backward, rightward and leftward when the leg is moved by the ligaments. Thus, it is preferable that the femur joint member and the bearing member be in contact with each other in various manners according to the curvature when the knee joint moves. This serves to properly disperse the stress.

The femur joint member 100 has a "U" shape and is made of a biocompatible material. The upper portion of the femur joint member 100 has a part for receiving the femur 1, and the lower portion thereof has a curved surface which has various curvatures and approximates a spherical shape. The femur joint member 100 includes a femur receiving part 110 and a locking protrusion 150. Part of the femur is cut, so that it is received by the femur receiving part of the femur joint member 100. The locking protrusion 150 couples the femur more firmly to the femur receiving part 110. Further, the femur joint member 100 has on its lower surface a curved contact portion 130 which is in contact with the bearing member 500 that will be described below.

The femur receiving part 110 is positioned at the interior of the upper portion of the femur joint member 100, and is firmly coupled to the incision surface of the lower portion of the femur. Preferably, the femur receiving part 110 has a rough surface or is made of a porous material so as to be firmly coupled to the femur.

The locking protrusion 150 extends upwards from the femur receiving part 110 to be inserted into the femur 1, and has the shape of a screw. More preferably, the locking protrusion 150 has a locking part which is shaped to be inserted into the femur and firmly grip the bone tissue of the femur. Further, the locking protrusion 150 may be designed in various shapes, for example, a detachable structure. It falls within the scope of the present invention.

When the femur moves while making contact with grooves 510 of the bearing member 500, the contact portion 130 is preferably shaped to maximally enlarge the contact area, thus naturally dispersing stress. Here, the curvature of the contact portion 130 when seen from the side is different from that of the contact portion when seen from the front. The different curvatures enable natural movement even when the knee joint moves forward and backward, and increases the contact area, thus dispersing stress. Further, when the knee joint is slightly abducted leftward or rightward or one contact portion is slightly lifted, a large stress is concentrated on the other contact portion. In this case, the different curvatures increase the contact area so as to disperse stress.

The tibia joint member 300 is fitted into and secured to the upper portion of the tibia in the typical artificial knee joint, is made of a biocompatible material, and supports the bearing member 500 which will be described below. Further, the tibia joint member may be formed in various types, and is typically classified into a mobile type and a fixed type according to the coupling relation between the tibia joint member and the bearing member. In the case of the mobile type tibia joint member, the bearing member 500 may rotate or move forward and backward on the tibia joint member 300, thus achieving the more natural movement of the knee joint. However, in the case of the fixed type tibia joint member, the bearing member 500 is fixed to the tibia joint member 300 so as not to be moved. The fixed type tibia joint member makes free movement difficult, but realizes stable movement owing to firm coupling. Thus, in the fixed type tibia joint member, the bearing member 500 may be integrated with the tibia joint member 300 into a single structure. In this case, it is natural that the effect realized by the bearing member 500 which is coupled to and makes contact with the femur joint member 100 be also achieved by the tibia joint member 300. Thus, the effect achieved by the components and operation of the bearing member 500 according to the present invention can be achieved by the tibia joint member 300. This falls within the scope of the present invention.

The bearing member 500 is the important part of the present invention, and is provided between the femur joint member 100 and the tibia joint member 300 to serve as the cartilage of the human body. Thus, the bearing member 500 is preferably made of polyethylene unlike the material of the femur joint member and the tibia joint member. Polyethylene does not produce impurities when it wears, and does not generate heat due to friction, so that it withstands friction well, and has a smooth surface to permit natural frictional contact. The bearing member 500 includes on its upper surface the grooves 510 which are in contact with the contact portion 130 of the femur joint member 100, and includes front and rear projecting parts 530 and 540 which are provided on the front and rear ends of the bearing member 500 when viewed from the side. Further, the bearing member 500 of the artificial knee joint according to the present invention may include the post 570 which serves as the posterior cruciate ligament of the PS type artificial knee joint which is implanted with the posterior cruciate ligament having been removed. FIG. 4 illustrates the bearing member of the CR type artificial knee joint which is implanted in the state in which the posterior cruciate ligament is retained, and FIG. 5 illustrates the bearing member of the PS type artificial knee joint which is implanted in the state in which the posterior cruciate ligament has been removed. As described above, the PS type bearing member of FIG. 5 includes the post 570.

The grooves 510 are portions which are depressed in both sides of the bearing member 500 according to the present invention. Friction occurs between the grooves and the contact portion, when the contact portion 130 of the femur joint member 100 makes contact with the grooves, so that the femur joint member 100 rotates. Thus, as the grooves are in contact with the contact portion, load is concentrated on the grooves, so that it is necessary to maximize a contact area so as to prevent the concentration of stress. Therefore, it is preferable that the grooves 510 have curvature corresponding to that of the contact portion 130 of the femur joint member 100. In the present invention, the grooves 510 provide movement similar to the movement of the natural knee, in addition to guaranteeing natural rolling. The conventional grooves are formed in a linear shape, but the grooves of the present invention are imparted with a curvature axis Y as shown in FIGS. 4 and 5, when viewed from the plane. As such, the grooves have the curvature axis Y, so that the artificial knee joint may slightly move leftward or rightward similarly to the movement of the natural knee when the knee is bent or stretched out during the rolling of the femur joint member 100 which is in contact with the upper surface of the bearing member. Actually, when the knee is moved to be bent, the tibia region tends to be adducted inward relative to the body. In contrast, when the knee is stretched out, the tibia region tends to be abducted outward relative to the body. The groove 510 includes a planar flexion and a side flexion. As shown in FIGS. 4 and 5, the planar flexion is the portion which has curvature on a plane when viewing the groove 510 from top. As shown in FIG. 10, the side flexion is the portion which has curvature in section when viewing the groove 510 from side. Since the planar and side flexions of the groove 510 are the important part of the present invention, they will be described below in detail.

Referring to FIGS. 4 to 9, the planar flexion includes planar front and rear flexions 511 and 512 having different curvatures. First, the terms planar front and rear flexions 511 and 512 will be explained. The planar front flexion means the portion which is in contact with the contact portion 130 of the femur joint member 100 when the knee is completely stretched out and thus is bent slightly forward as shown in FIG. 8, and generally corresponds to the front portion of the groove 510 of the bearing member 500. Next, the term planar rear flexion 512 will be explained; when the knee is fully bent back as shown in FIG. 6, the planar rear flexion 512 is the portion which is in contact with the contact portion 130 of the femur joint member 100, and generally corresponds to the rear portion of the groove 510 of the bearing member 500. FIG. 7 and 9 show the planar rear flexion 512 and the planar front flexion 511. Since the start axis of the drawings is merely the axis representing the radius of curvature, it is apparent that the start axis does not show the definite positions of the planar front and rear flexions 511 and 512. That is, as described above, the planar front and rear flexions 511 and 512 are determined by the portion which is in contact with the contact portion 130 according to the bending angle of the femur joint member 100. The reason why the planar front flexion 511 and the planar rear flexion 512 have different curvatures is because an adduction angle when the natural knee is bent is different from an abduction angle because the knee is bent slightly forward when the knee is completely stretched out. Especially, it is preferable that the curvature radius RB of the planar rear flexion 512 of FIG. 7 be larger than the curvature radius RA of the planar front flexion 511 of FIG 9. Such a construction allows the knee joint to be adducted and abducted at different angles when the knee joint is bent inward (see FIG. 6) or is stretched out to be bent slightly outward (see FIG. 8) in the state in which the artificial knee joint of the present invention is implanted, as shown in FIGS. 6 and 8. Thereby, the curvature radius RA of the planar front flexion is smaller than the curvature radius RB of the planar rear flexion. The reason is because roll back occurs when the knee is bent, the femur moves a longer distance, and the distance moved increases as the radius increases at the same axial rotating angle of the femur. Referring to FIGS. 6 and 7, the bearing member 500 of the present invention is formed such that the curvature radius RB of the planar rear flexion 512 of FIG. 7 is larger than the curvature radius RA of the planar front flexion 511, because the adduction angle when the knee is bent inward as shown in FIG. 6 is smaller. Further, as shown in FIGS. 8 and 9, the bearing member 500 of the present invention is formed such that the curvature radius RA of the planar front flexion 511 of FIG. 9 is smaller than the curvature radius RB of the planar rear flexion 512, because an abduction angle when the knee is stretched forward as shown in FIG. 8 is larger. The reason why the adduction angle is different from the abduction angle as such is because the distance moved becomes longer as the radius increases at the same axial rotating angle of the femur. The different curvature radii RA and RB of the planar front and rear flexions 511 and 512 enable the knee to be more naturally moved.

Hereinafter, the side flexion of the groove 510 of the bearing member 500 will be described in detail with reference to FIG. 10. The side flexion is the portion having a predetermined curvature when viewing the bearing member 500 from the side, and includes side front and rear flexions 515 and 516 having different curvature radii as shown in FIG. 10. Similarly to the above-mentioned planar front and rear flexions 511 and 512, the side front and rear flexions 515 and 516 are determined according to what part of the contact portion 130 of the femur joint member 100 is in contact with the groove 510 of the bearing member 500 when the knee joint is bent or stretched out. Thus, when seen from a sectional view, the side front flexion 515 is located at the front portion of the groove 510 of the bearing member 500, and the side rear flexion 516 is located at the rear portion of the groove 510 of the bearing member 500. As shown in FIG. 10, the side front flexion 515 has the curvature radius of R1 along the arc a-a', and the side rear flexion 516 has the curvature radius of R2 along the arc b-b'. The side front flexion 515 has the curvature radius R1 which is smaller than that of the side rear flexion 516. Thereby, when the contact portion 130 of the femur joint member 100 comes into contact with the groove 510 of the bearing member 500, the side front flexion 515 has the small curvature radius R1, thus preventing anterior dislocation. The side rear flexion 516 has the larger curvature radius R2, thus allowing the femur joint member 100 to be bent much more, therefore enabling sufficient roll back. A difference in curvature between the side front and rear flexions 515 and 516 corresponds to a difference in height between the front and rear projecting parts 530 and 540 of the bearing member 500, thus providing natural knee movement and effectively preventing the dislocation of the knee joint.

As shown in FIGS. 10 to 12, the front and rear projecting parts 530 and 540 have different heights from a reference horizontal axis C, that is, the height H1 of the front projecting part and the height H2 of the rear projecting part. The front projecting part 530 is formed to be higher than the front projecting part of the conventional bearing member, and the rear projecting part 540 is formed to be lower than the rear projecting part of the conventional bearing member. This enables natural knee movement and prevents anterior dislocation, similarly to a difference in curvature between the side front and rear flexions 515 and 516. That is, when viewing the bearing member 500 from the side, the front and rear projecting parts 530 and 540 are portions which are projected from front and rear portions to predetermined heights. Especially, the height H1 of the front projecting part 530 is set to be high, thus preventing the femur joint member 100 from being separated from the bearing member 500 when a patient having the artificial knee joint of the present invention bends his or her knee at a large angle. The height H2 of the rear projecting part 540 is set to be low, thus preventing dislocation when the knee is bent at a large angle and rolls back, and allowing the knee joint to be stably moved.

The post 570 is provided only in the PS type of FIG. 5, and is formed when the PS type artificial knee joint is implanted in the state in which the posterior cruciate ligament has been removed. The post 570 is required to prevent anterior dislocation and provide the natural movement of the knee when the artificial knee joint is bent, in place of the posterior cruciate ligament. In this case, although not shown in the drawings, the femur joint member 100 includes a cam which engages with the post 570. As such, the post 570 of the bearing member 500 and the cam of the femur joint member 100 perform the function of the posterior cruciate ligament of the PS type. Since the post and the cam of the PS type are known to those skilled in the art, the detailed description will be omitted.

The operation and use of the artificial knee joint according to the preferred embodiment of the present invention will be described below in detail with reference to the accompanying drawings.

FIG. 6 is a view illustrating the state in which the knee is bent in the artificial knee joint of the present invention, FIG. 7 is a view illustrating the planar rear flexion in the bearing member of the present invention, FIG. 8 is a view illustrating the state of the knee being stretched in the artificial knee joint of the present invention, FIG. 9 is a view illustrating the planar front flexion in the bearing member of the present invention, and FIG. 10 is a view illustrating the side flexion in the bearing member of the present invention.

The operation of the artificial knee joint according to the present invention will be described with reference to FIGS. 6 to 9. The bearing member 500 of the present invention includes the planar front and rear flexions 515 and 516 so as to simulate the movement of the natural knee. First, referring to FIGS. 6 and 7, when the artificial knee joint of the present invention is implanted and thereafter the knee is bent inward (see FIG. 6), the contact portion 130 of the femur joint member 100 is in contact with the planar rear flexion 512 of the groove 510 of the bearing member 500 and rolls. When the rolling movement is performed, as shown in FIG. 7, the planar rear flexion 512 of the groove 510 of the bearing member has a larger curvature radius RB. Thus, although the knee is bent by a comparatively large degree, the adduction caused by the bending may be similar to that of the natural knee. Further, when the natural knee is fully stretched out, as shown in FIG. 8, the knee is bent slightly forward. When the forward bending occurs, the abduction of the knee occurs. In this case, as shown in FIG. 9, the planar front flexion 511 of the groove 510 of the bearing member has a smaller curvature radius RA so that the abduction occurs when the knee is bent.

Hereinafter, the side flexion and the front and rear projecting parts which provide natural knee movement and sufficient roll back and prevent anterior dislocation will be described with reference to FIGS. 10 to 12. As shown in FIG. 10, the side flexion includes the side front and rear flexions 515 and 516 which have different curvatures. The side front flexion 515 has a curvature radius smaller than that of the side rear flexion 516, thus having a steeper slope. Thereby, when the knee is bent back, more stable movement is possible and anterior dislocation is prevented. In contrast, the side rear flexion 516 has a larger curvature radius, so that a slope becomes gentle, thus providing sufficient roll back in the flection of the knee. Further, a difference in curvature radius between the side front and rear flexions 515 and 516 leads to a difference in height between the front and rear projecting parts 530 and 540. The height of the front projecting part 530 is H1 and is larger than the height H2 of the rear projecting part 540. The high height H1 of the front projecting part 530 prevents anterior dislocation when the knee is bent forward or fully stretched out. The low height H2 of the rear projecting part 540 allows the artificial knee joint of the present invention to be bent inward by a larger angle. Meanwhile, if the rear projecting part 540 is formed to be high, the femur region to which there is no femur joint member 100 joined may come into contact with and damage the rear projecting part 540, at the time of the roll back. In order to solve the problem, the rear projecting part is formed to be low.

Although the preferred embodiments of the present invention have been disclosed for illustrative purposes, those skilled in the art will appreciate that various modifications, additions and substitutions are possible, without departing from the scope of the invention as disclosed in the accompanying claims.

## Claims

1. A bearing member (500) for an artificial knee joint, comprising:
a groove (510) provided on an upper surface thereof and having a planar flexion to move a knee joint similarly to a real knee movement; and
a side flexion including a side front flexion (515) and a side rear flexion (516) which have different curvature radii so as to more naturally move the knee joint,
wherein the planar flexion comprises a planar rear flexion (512) which is a contact portion when a knee is bent backward, and a planar front flexion (511) which is a contact portion when the knee is stretched forward,
**characterized in that**
a curvature radius of the planar front flexion (511) is smaller than a curvature radius of the planar rear flexion (512).

2. The bearing member (500) according to claim 1, further comprising:
a front projecting part (530) and a rear projecting part (540) being provided on the front and rear ends of the bearing member (500) when viewed from the side.

3. The bearing member (500) according to claim 2,
wherein a height of the front projecting part (530) is higher than a height of the rear projecting part (540), thus allowing a bending movement of the knee to be more naturally performed when the knee is bent, and preventing anterior dislocation of a femur joint member (100).

4. The bearing member (500) according to any one of claims 1 to 3, wherein the curvature radius of the side front flexion (515) is smaller than the curvature radius of the side rear flexion (516).

5. An artificial knee joint comprising a bearing member (500) according to any one of claims 1 to 4 including a flexion comprising a femur joint member (100) attached to an end portion of a femur (1), a tibia joint member (300) attached to an end portion of a tibia (3), and the bearing member (500) positioned between the femur joint member (100) and the tibia joint member (300).

## Patentansprüche

1. Lagerelement (500) für ein künstliches Kniegelenk, welches Folgendes aufweist:
eine Vertiefung (510), die an einer oberen Oberfläche hiervon vorgesehen ist, und die eine ebene Flexion besitzt, um ein Kniegelenk ähnlich wie eine echte Kniebewegung zu bewegen; und
eine Seiten-Flexion mit einer Seiten-Frontal-Flexion (515) und einer Seiten-Rückseiten-Flexion (516), die unterschiedliche Krümmungsradien besitzen, um das Kniegelenk natürlicher zu bewegen,
wobei die ebene Flexion eine ebene Rückseiten-Flexion (512) aufweist, die ein Kontaktabschnitt ist, wenn das Knie rückwärts gebogen ist, und eine ebene Frontal-Flexion (511), die ein Kontaktabschnitt ist, wenn das Knie nach vorne gestreckt ist,
**dadurch gekennzeichnet, dass**
der Krümmungsradius der ebenen Frontal-Flexion (511) kleiner ist als der Krümmungsradius der ebenen Rückseiten-Flexion (512).

2. Lagerelement (500) nach Anspruch 1, welches ferner aufweist:
einen frontalen vorstehenden Teil (530) und einen rückseitigen vorstehenden Teil (540), die - seitlich betrachtet - auf der Vorderseite und der Rückseite des Lagerelements (500) vorgesehen sind.

3. Lagerelement (500) nach Anspruch 2,
bei welchem eine Höhe des frontalen vorstehenden Teil (530) höher ist als eine Höhe des rückseitigen vorstehenden Teils (540), wodurch eine beugende Bewegung des Knies natürlicher durchgeführt werden kann, wenn das Knie gebeugt wird, und wobei eine anteriore Dislozierung eines femoralen Gelenkelements (100) vermieden wird.

4. Lagerelement (500) nach einem der Ansprüche 1 bis 3, bei welchem der Krümmungsradius der Seiten-Front-Flexion (515) kleiner ist als der Krümmungsradius der Seiten-Rückseiten-Flexion (516).

5. Künstliches Kniegelenk, welches ein Lagerelement (500) mit einer Flexion nach einem der Ansprüche 1 bis 4 aufweist, welches künstliche Kniegelenk ein femorales Gelenkelement (100) aufweist, das an einem Endabschnitt eines Femurs (1) angebracht ist, ein tibiales Gelenkelement (300), das an einen Endabschnitt einer Tibia (3) angebracht ist, wobei das Lagerelement (500) zwischen dem femoralen Gelenkelement (100) und dem tibialen Gelenkelement (300) positioniert ist.

## Revendications

1. Élément support (500) pour une articulation artificielle du genou, comprenant :
une rainure (510) prévue sur une surface supérieure de celui-ci et ayant une courbure plane pour déplacer une articulation du genou de manière similaire à un mouvement du genou réel ; et
une courbure latérale comportant une courbure avant latérale (515) et une courbure arrière latérale (516) qui présentent différents rayons de courbure de manière à déplacer l'articulation du genou plus naturellement,
la courbure plane comprenant une courbure arrière plane (512) qui est une portion de contact lorsqu'un genou est fléchi vers l'arrière, et une courbure avant plane (511) qui est une portion de contact lorsque le genou est étiré vers l'avant, **caractérisé en ce**
**qu'**un rayon de courbure de la courbure avant plane (511) est inférieur à un rayon de courbure de la courbure arrière plane (512).

2. Élément support (500) selon la revendication 1, comprenant en outre :
une partie saillante avant (530) et une partie saillante arrière (540) prévues sur les extrémités avant et arrière de l'élément support (500) lorsqu'il est vu de côté.

3. Élément support (500) selon la revendication 2,
dans lequel une hauteur de la partie saillante avant (530) est supérieure à une hauteur de la partie saillante arrière (540), permettant ainsi d'effectuer plus naturellement un mouvement de flexion du genou lorsque le genou est fléchi, et empêchant une luxation antérieure d'un élément d'articulation du fémur (100).

4. Élément support (500) selon l'une quelconque des revendications 1 à 3, dans lequel le rayon de courbure de la courbure avant latérale (515) est inférieur au rayon de courbure de la courbure arrière latérale (516).

5. Articulation artificielle du genou comprenant un élément support (500) selon l'une quelconque des revendications 1 à 4 comportant une courbure comprenant un élément d'articulation du fémur (100) fixé à une portion d'extrémité d'un fémur (1), et un élément d'articulation du tibia (300) fixé à une portion d'extrémité d'un tibia (3), et l'élément support (500) positionné entre l'élément d'articulation du fémur (100) et l'élément d'articulation du tibia (300).
